# EUROPEAN PATENT SPECIFICATION

(11) **EP 4 469 120 B1**
(45) Date of publication and mention of the grant of the patent: **04.03.2026**
(21) Application number: 23702912.9
(22) Date of filing: 24.01.2023
(51) Int. Cl.: A61M 25/00, A61M 25/06

(54) **EXPANDABLE INTRODUCER SHEATH**
EXPANDIERBARE EINFÜHRUNGSHÜLSE
GAINE D'INTRODUCTION EXTENSIBLE

(30) Priority: 28.01.2022 US 202263304073 P
(43) Date of publication of application: 04.12.2024
(73) Proprietor: Medtronic, Inc., Minneapolis, MN 55432 (US)
(72) Inventor: ANDERSON, Marc A., Galway (IE); CLARKE, Luke A., Galway (IE)
(74) Representative: Zimmermann & Partner Patentanwälte mbB
(86) International application number: PCT/IB2023/050580
(87) International publication number: WO 2023/144693

(56) References cited:
- EP-A1- 3 280 479
- WO-A1-2021/226421
- US-A1- 2008 188 928
- US-A1- 2018 161 064
- US-A1- 2019 321 175

## Description

### FIELD OF THE INVENTION

The present invention relates to an introducer device for providing percutaneous access to a patient's vasculature for a transcatheter device delivery system, and, more particularly to an expandable introducer device having a folded liner within an expandable introducer sheath for accommodating passage of the transcatheter device delivery system therethrough.

### BACKGROUND

An introducer device is used to provide percutaneous access to the vascular system of a patient and functions to permit the introduction and positioning of various minimally invasive delivery devices within the patient's vasculature. In general, having the smallest insertion profile is the most desirable and leads to less complications, less trauma, and improved patient outcomes. Some delivery devices, however, are large and require large sheaths to accommodate and help deliver them to the desired site within the body. US 2019/0321175 A1 relates to an expandable sheath with elastomeric cross sectional portions.

### BRIEF SUMMARY OF THE INVENTION

The invention is defined in the claims. In accordance with an aspect hereof, an expandable introducer is disclosed. The expandable introducer includes an introducer hub, an introducer sheath coupled to the introducer hub and extending distally therefrom. The introducer sheath includes a mesh tube, a liner disposed inside the mesh tube, the liner defining a central lumen, and an axial strip disposed between the liner and the mesh tube, the axial strip bonding the liner to the mesh tube, wherein the introducer sheath is radially expandable from a radially unexpanded state wherein the central lumen has a first diameter to a radially expanded state wherein the central lumen has a second diameter larger than the first diameter.

In another aspect hereof, and in combination with any other aspects, the disclosure provides the liner in the radially unexpanded state includes a fold, and wherein the liner unfolds to expand to the radially expanded state.

In another aspect hereof, and in combination with any other aspects, the disclosure provides the first diameter is in the range of about 3.0 mm to about 5.0 mm.

In another aspect hereof, and in combination with any other aspects, the disclosure provides the second diameter is in the range of about 8.0 mm to about 10.0 mm.

In another aspect hereof, and in combination with any other aspects, the disclosure provides the mesh tube comprises a braided, mesh material comprising a plurality of braided fibers arranged in a braided or mesh pattern to form an open-cell fibrous wall material.

In another aspect hereof, and in combination with any other aspects, the disclosure provides the plurality of braided fibers comprise a polymeric material.

In another aspect hereof, and in combination with any other aspects, the disclosure provides the liner is a thin, flexible plastic tube configured to expand from a folded state to an unfolded state in response to the device being advanced through the central lumen.

In another aspect hereof, and in combination with any other aspects, the disclosure provides the liner comprises a single fold that is configured to unfold to radially expand the liner as the device is advanced through the central lumen.

In another aspect hereof, and in combination with any other aspects, the disclosure provides the liner comprises two or more folds that are configured to unfold to radially expand the liner as the device is advanced through the central lumen.

In another aspect hereof, and in combination with any other aspects, the disclosure provides the liner comprises polytetrafluoroethylene (PTFE), expanded polytetrafluoroethylene (ePTFE), fluorinated ethylene propylene (FEP), and/or perfluoroalkoxy (PFA).

In another aspect hereof, and in combination with any other aspects, the disclosure provides the liner has a thickness in the range of about 0.05 mm to about 0.40 mm.

In another aspect hereof, and in combination with any other aspects, the disclosure provides the axial strip comprises a high durometer polymer.

In another aspect hereof, and in combination with any other aspects, the disclosure provides the axial strip comprises a polyamide 12 material and/or a polyether block amide material.

In another aspect hereof, and in combination with any other aspects, the disclosure provides the axial strip extends about 70° to about 180° around a circumference of the mesh tube with the introducer sheath in the radially unexpanded state.

In another aspect hereof, and in combination with any other aspects, the disclosure provides the axial strip includes an axial spine.

In another aspect hereof, and in combination with any other aspects, the disclosure provides the axial spine comprises a thin, rigid wire.

In another aspect hereof, and in combination with any other aspects, the disclosure provides the axial spine comprises two or more thin, rigid wires.

In another aspect hereof, and in combination with any other aspects, the disclosure provides the introducer sheath includes a proximal portion coated with a substantially non-porous expandable layer to assist with establishing a hemostatic seal when the expandable introducer is introduced to a patient's vasculature.

In one aspect hereof, and in combination with any other aspects, the present disclosure provides a method (not claimed) of tracking the expandable introducer into a vessel, the expandable introducer includes an introducer hub and an introducer sheath, the introducer sheath including a mesh tube, a liner disposed inside the mesh tube, the liner defining a central lumen, and an axial strip disposed in between the liner and the cylindrical mesh, the axial strip connecting the liner and the mesh tube to one another, advancing the expandable introducer until a distal end of the introducer hub is snug against a vessel to establish a hemostatic seal, inserting a device through a proximal end of the expandable introducer, and advancing the device through the central lumen of the liner.

In another aspect hereof, and in combination with any other aspects, the disclosure provides the introducer sheath expands radially in response to the device being advanced through the central lumen.

In another aspect hereof, and in combination with any other aspects, the disclosure provides expanding the introducer sheath by injecting saline into the lumen prior to advance the device through the lumen.

The details of one or more aspects of the disclosure are set forth in the accompanying drawings and the description below. Other features, objects, and advantages of the techniques described in this disclosure will be apparent from the description and drawings, and from the claims.

In one aspect hereof, and in combination with any other aspects, the present disclosure provides a method (not claimed) for manufacturing the introducer sheath, the method comprising, loading the introducer sheath on a mandrel, the introducer sheath including a mesh tube, a liner disposed inside the mesh tube, and an axial strip disposed between the liner and the mesh tube, utilizing a heat shrink wrap to bond the axial strip to the liner and the mesh tube, allowing the introducer sheath to cool and harder, and removing the introducer sheath from the mandrel.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings may not be to scale.
FIG. 1 shows an expandable introducer according to an embodiment hereof.
FIG. 2A shows an assembled expandable introducer sheath of the expandable introducer of FIG. 1 according to an embodiment hereof.
FIG. 2B shows a mesh tube of the expandable introducer sheath of FIG. 2A according to an embodiment hereof.
FIG. 2C shows a liner of the expandable introducer sheath of FIG. 2A according to an embodiment hereof.
FIG. 2D shows an axial strip of the expandable introducer sheath of FIG. 2A according to an embodiment hereof.
FIG. 3A shows an axial strip with one axial spine according to an embodiment hereof.
FIG. 3B shows an axial strip with two axial spines according to an embodiment hereof.
FIG. 4A shows the expandable introducer sheath with one axial strip in an unexpanded state according to an embodiment hereof.
FIG. 4B shows a cross-section taken along line B-B of FIG. 4A.
FIG. 5A shows the expandable introducer sheath of FIG. 4A in an expanded state according to an embodiment hereof.
FIG. 5B shows a cross-section taken along line B-B of FIG. 5A.
FIG. 6A shows an expandable introducer sheath with two axial strips in an unexpanded state according to an embodiment hereof.
FIG. 6B shows a cross-section taken along line B-B of FIG. 6A.
FIG. 7A shows the expandable introducer sheath of FIG. 6A in an expanded state according to an embodiment hereof.
FIG. 7B shows a cross-section taken along line B-B of FIG. 7A.
FIG. 8A shows an expandable introducer sheath with two folds in the liner in an unexpanded state according to an embodiment hereof.
FIG. 8B shows a cross-section taken along line B-B of FIG. 8A.
FIG. 9 is a block diagram that shows a method of using the expandable introducer according to an embodiment hereof.
FIG. 10 shows a step in the method of FIG. 9, showing a guidewire positioned in the vessel of a patient according to an embodiment hereof.
FIG. 11 shows a step in the method of FIG. 9, showing the expandable introducer in the vessel of a patient according to an embodiment hereof.
FIG. 12 shows a step in the method of FIG. 9, showing a device being advanced through the expandable introducer in the vessel of a patient according to an embodiment hereof.
FIG. 13 shows the expandable introducer in the vessel of a patient according to an embodiment hereof.

### DETAILED DESCRIPTION

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components associated with, for example, a medical device. The following detailed description is merely exemplary in nature and is not intended to limit the invention of the application and uses of the invention. Furthermore, there is no intention to be bound by any expressed or implied theory presented in the preceding field of the invention, background, summary or the following detailed description.

As used in this specification, the singular forms "a", "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%. It should be understood that use of the term "about" also includes the specifically recited number of value.

The terms "proximal" and "distal" herein are used with reference to the clinician using the devices. Therefore, "proximal" and "proximally" mean in the direction toward the clinician, and "distal" and "distally" mean in the direction away from the clinician.

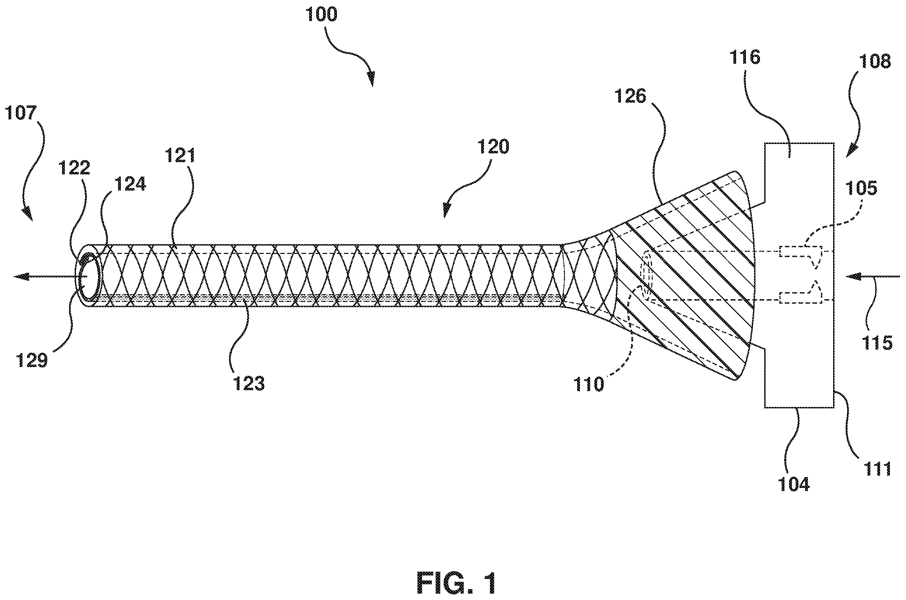

As shown in FIG. 1, an expandable introducer 100 includes an introducer hub 104 and an introducer sheath 120. The expandable introducer 100 includes distal end 107 and a proximal end 108. The introducer sheath 120 is coupled to a distal portion 110 of the introducer hub 104 and extends distally therefrom.

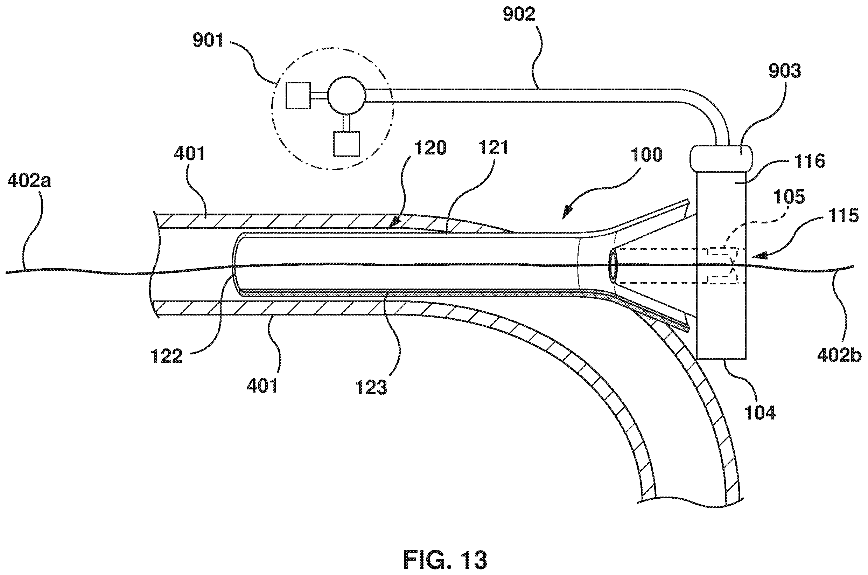

The introducer hub 104 includes a proximal end 111, a distal end 110, and a passageway 115 extending from the proximal end 111 to the distal end 110 of the introducer hub 104. The passageway 115 is sized and shaped to accommodate a device 900 therethrough, as described in more detail below. The introducer hub 104 further includes a seal 105 disposed within the passageway 115 to seal around a dilator shaft of a dilator (not shown) and or the device 900, and prevent blood flow out of a patient's vessel when the expandable introducer 100 is in use. The introducer hub 104 further includes an outer surface 116 tapered in a distal direction such that the distal end 110 of the introducer hub 104 has a first outer diameter of the distal end 110 that is smaller than a second outer diameter of the proximal end 111 of the introducer hub 104. Although the passageway 115 in FIG. 1 is shown have a generally constant diameter, in other embodiments the passageway 115 may be tapered. In such embodiments, the distal end 110 of the introducer hub 104 may have a first inner diameter (diameter of the passageway 115) that is smaller than a second inner diameter (diameter of the passageway 115) of the proximal end 111 of the introducer hub 104. In an example, the first outer diameter of the introducer hub 104 is about 10 mm to about 20 mm, the second outer diameter of the introducer hub 104 is about 20 mm to about 35 mm, the first inner diameter of the introducer hub 104 is about 5 mm to about 15 mm, and the second inner diameter of the introducer hub 104 is about 15 mm to 30 mm. In some embodiments, the introducer hub 104 includes a side port 903 which may be utilized for infusing saline or other fluids into a central lumen 129 of the introducer sheath 120, as shown and described with respect to FIG. 13.

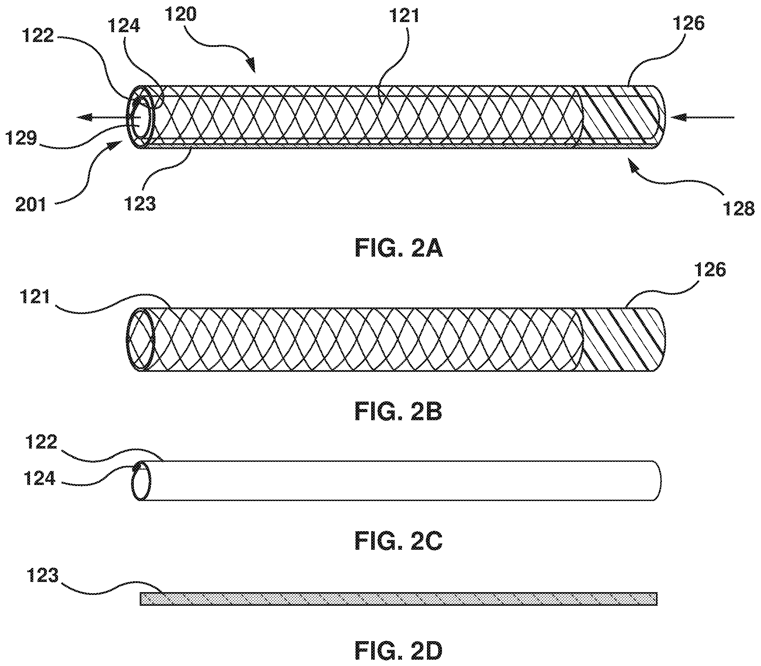

FIG. 2A shows the introducer sheath 120 according to embodiments hereof. The introducer sheath 200 includes a mesh tube 121, a liner 122, and an axial strip 123, as shown in FIGS. 2B-2D.

The mesh tube 121, shown in FIGS. 2A and 2B, is the outermost layer of the introducer sheath 120. The mesh tube 121 is a thin, flexible braided tube configured to expand in response to a device 900 being advanced therethrough. The mesh tube 121 may be a braided, mesh material. The mesh tube 121 may include a plurality of braided fibers arranged in a braided or mesh pattern to form an open-cell fibrous wall material. The fibers may be a polymeric material, such as polyester, polyamide, polypropylene, or copolymers thereof. The mesh tube 121 may be as described in U.S. Patent Publication No. 2019/0183525 A9, which is a corrected publication of U.S. Publication No. 2016/0128723 A1**.** The mesh tube 121 may include a proximal portion 126 that is coated with a substantially non-porous expandable layer to assist with establishing a hemostatic seal when the expandable introducer 100 is introduced to a patient's vasculature. The mesh tube 121 may have a wall thickness in the range of about 0.4-1.0 mm.

The liner 122, as shown in FIGS. 2A and FIG. 2C, is a thin, difficult to pierce, low-friction plastic tube disposed inside an interior surface of the mesh tube 121. The liner 122 is expandable via folds 124, as described in more detail below, and is configured to prevent the device 900 from snagging or catching on the mesh tube 121 of the introducer sheath 120 when being advanced or retracted through the introducer sheath 120. The liner 122 is configured to be in a folded, unexpanded state when inserted into the vessel of a patient, and to expand to an unfolded, expanded state in response to the device 900 being advanced distally therethrough. In embodiments, the liner 122 can include a single fold 124 when in a folded, unexpanded state. In other embodiments, the liner 122 can include two or more folds 124, or three or more folds 124 when in the folded, unexpanded state. The liner 122 may be a formed from polytetrafluoroethylene (PTFE), expanded polytetrafluoroethylene (ePTFE), fluorinated ethylene propylene (FEP), perfluoroalkoxy (PFA), or other materials known to those skilled in the art suitable for the purposes described herein, or any combination thereof. The liner 122 may have a wall thickness in the range of about 0.05-0.40 nun.

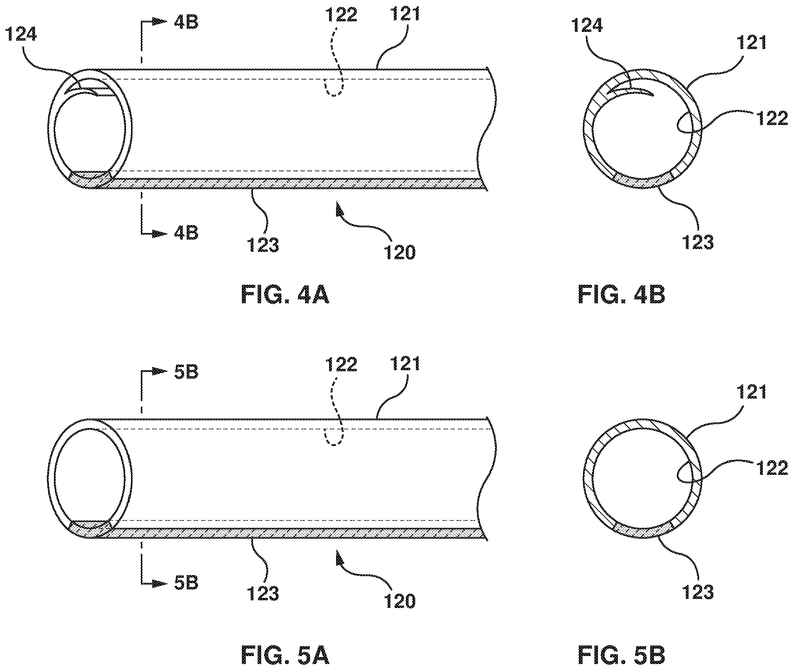

The axial strip 123, shown in FIGS. 2A and 2D, is disposed radially between the liner 122 and the mesh tube 1121. The axial strip 123 is configured to couple the liner 122 to the mesh tube 121. The axial strip 123 may extend along an entire longitudinal length of the introducer sheath 120, *i.e.,* from the distal-most end of the introducer sheath 120 to the proximal-most end of the introducer sheath 120. The axial strip 123 may extend about 70°-180° around a circumference of the introducer sheath 200 in a radially unexpanded state, as shown in FIG. 4B. In other embodiments, the axial strip 123 may extend about 80°-150°, or 90°-130°, or 100°-120° around the circumference of the introducer sheath 120 in the radially unexpanded state. The axial strip 123 may be made of a high durometer polymer, such as, but not limited to, a polyamide 12 material (such as VESTAMID), a polyether block amide (such as PEBAX), pellethanes, urethanes, or other materials with similar characteristics known to those skilled in the art, or combinations thereof. The axial strip 123 may be attached to both the liner 122 and the mesh tube 121 via a heat shrink wrap, as explained in more detail below, adhesives, or other suitable attachment devices and methods.

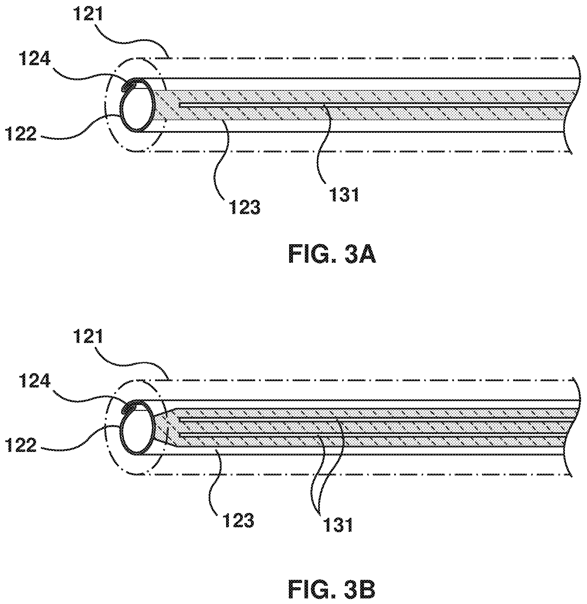

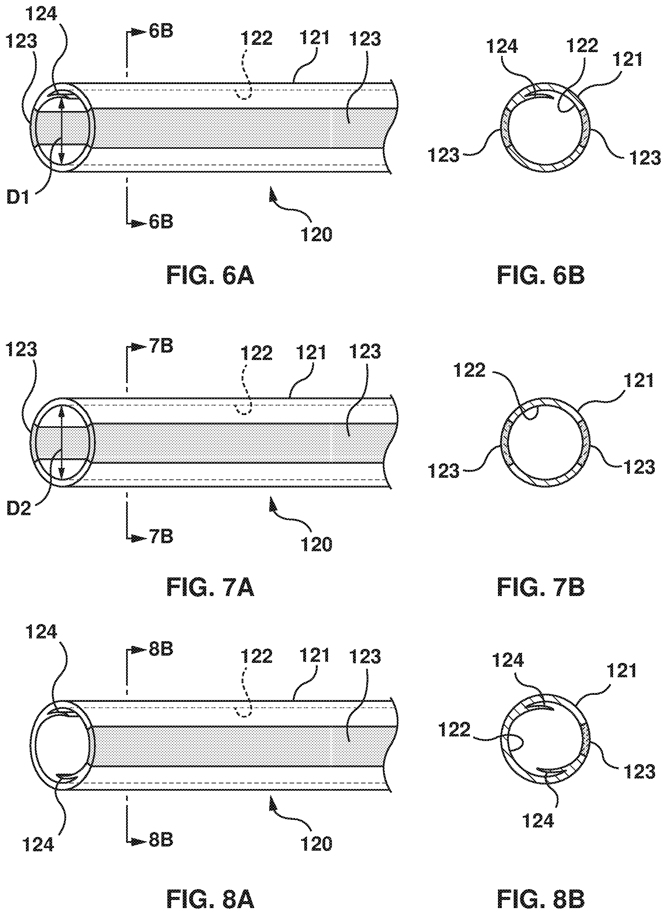

In some embodiments, the introducer sheath 120 may include two axial strips 123, as best shown in FIGS. 6A-7B. For example, and not by way of limitation, the two axial strips 123 may be disposed 180 degrees apart from each other. In other embodiments, three axial strips may couple the liner 122 to the mesh tube 121 and be disposed 120 degrees apart from each other, or four axial strips 123 may couple the liner 122 and the mesh tube 121 and be 90 degrees apart from each other, of any number of suitable axial strips 123 may couple the liner 122 and the mesh tube 121 and distributed around the central axis of the thereof at an appropriate spacing. In some embodiments, the axial strip 123 may contain an axial spine 131, as shown in FIG. 3A. The axial spine 131 may be a single, thin rigid wire configured to support the structure of the introducer sheath 120. In other embodiments, the axial strip 123 may include two or more axial spines 131, as shown in FIG. 3B, or three or more axial spines 131

The liner 122 includes one fold 124 when in the folded, unexpanded state, as shown in FIG. 4A. FIG. 4B is a cross-section taken along line B-B of FIG. 4A that shows one fold 124 in the liner 122 and one axial strip 123 disposed between and coupling the liner 122 and the mesh tube 121 of the introducer sheath 120. FIG. 5A shows introducer sheath 120 in a radially expanded state with the liner 122 unfolded and the mesh tube 121 radially expanded. FIG. 5B is a cross-section taken along line B-B of FIG. 5A showing the liner 122 in the unfolded, expanded state, the mesh tube 121 in the radially expanded state, and the axial strip 123 disposed radially between and coupling the liner 122 and the mesh tube 121. Thus, as can be seen, central lumen 129 of the introducer sheath 120 has a first diameter D1 in a folded, unexpanded state that is smaller than a second diameter D2 of the central lumen 129 in an unfolded, expanded state. The first diameter of the introducer sheath 120 in the folded, unexpanded state may be in the range of about 3 mm to about 5mm. The second diameter of the introducer sheath 120 in the unfolded, expanded state may be in the range of about 8 mm to about 10 mm.

As explained above, the introducer sheath 120 includes one or more one axial strips 123 and the liner 122 includes one or more folds 124. FIGS. 6A and 6B show an example of an introducer sheath 120 with two axial strip 123 and one fold in the liner 122, in the folded, unexpanded state. FIG. 6B is a cross-section taken along line B-B of FIG. 6A showing one fold in the liner 122 with two axial strips 123 coupling the liner 122 and the mesh tube 121 of the introducer sheath 200. FIG. 7A shows the introducer sheath 120 of FIG. 6A in the unfolded, expanded state. FIG. 7B is a cross-section taken along line B-B of FIG. 7A. As can be seen in FIGS. 7A and 7B, the mesh tube 121 has radially expanded and the liner 122 has unfolded to radially expand.

FIG. 8A shows an introducer sheath 120 with a single axial strip 123 disposed radially between and coupling the liner 122 and the mesh tube 121, and two folds 124 in the liner 122. FIG. 8A shows the introducer sheath 120 with a single axial strip 123 and two folds 124 with the introducer 120 in the folded, unexpanded state. FIG. 8B is a cross-section taken along line B-B of FIG. 8A showing the two folds 124 in the liner 122 and the single axial strip 123 coupling the liner 122 and the mesh tube 121. When radially expanded, the mesh tube 121 will radially expand as explained above, and the liner 122 will radially expand via unfolding of the folds 124.

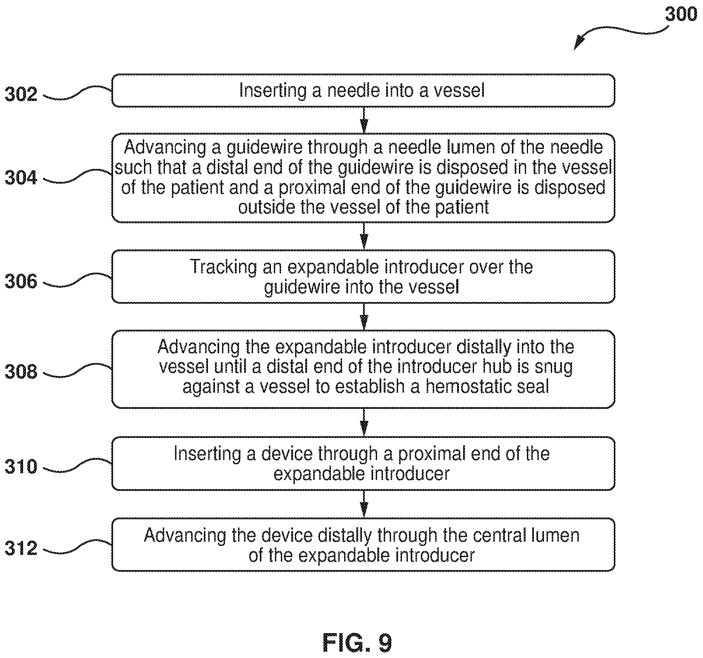

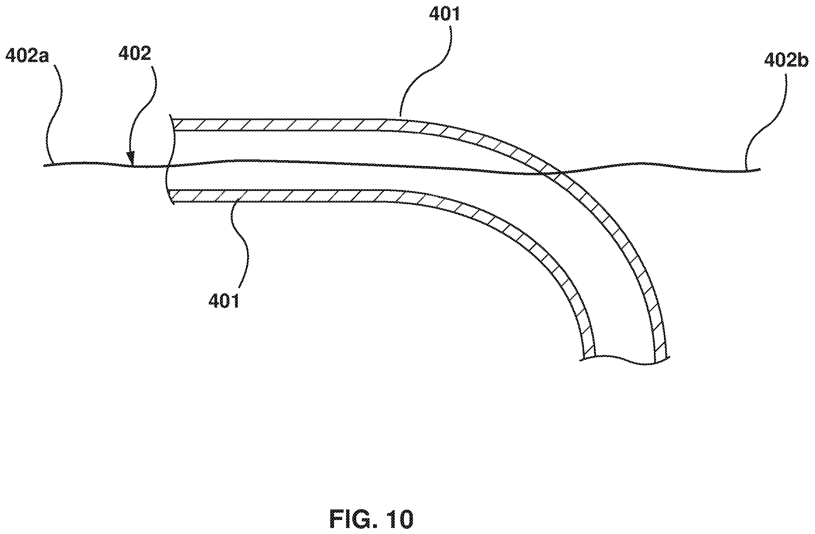

FIGS. 9-13 illustrate an embodiment of a method (not claimed) for accessing the vasculature of a patient and delivering the device 900 towards a desired site in the vasculature using the expandable introducer 100. The device 900 can be, for example, a delivery catheter for delivering a medical device, such as a heart valve prosthesis, to a treatment site, such as a native heart valve. In a step 302 of the method 300, a needle may be inserted through a wall of a vessel 401, wherein the needle includes a needle lumen. In a step 304 of the method 300, with the needle having gained access to the vessel, a guidewire 402 is inserted through an opening in a proximal end of the needle lumen. A distal end 402a of the guidewire 402 is advanced through the needle lumen and exits through a distal end of the needle lumen. Thus, a distal end 402a of the guidewire 402 is disposed in the vessel 401 of the patient and a proximal end 402b of the guidewire 402 is disposed outside of the patient, as shown in FIG. 10.

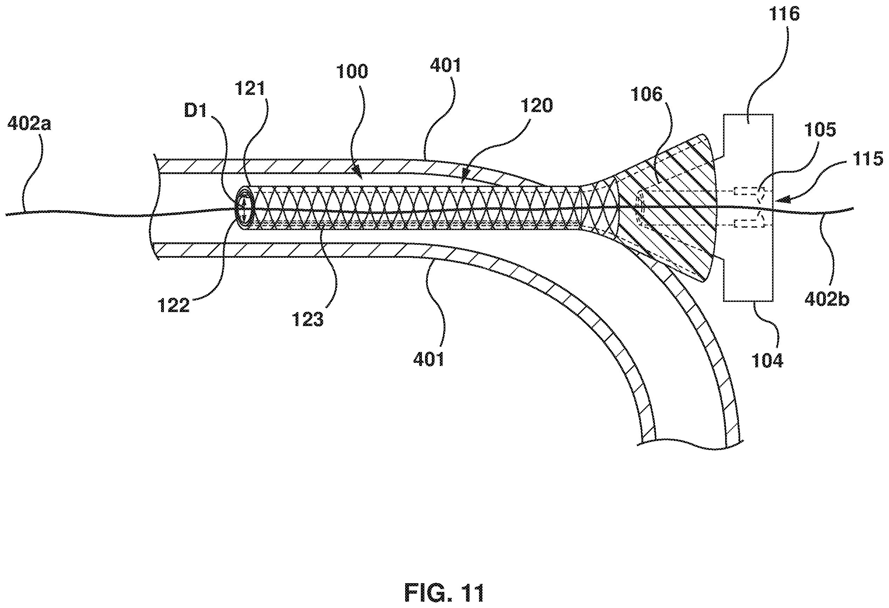

In a step 306 of the method 300, the proximal end 402b of the guidewire 402 is inserted through the expandable introducer 100 by entering the central lumen 129 of the introducer sheath 120 at the distal end 107 of the expandable introducer 100. The expandable introducer 100 is advanced distal over the guidewire 402 such that the guidewire extends from its distal end 402a through the central lumen 109 of the introducer sheath 120, through the passageway 115 of the introducer hub 104, and exits through an opening at the proximal end 108 of the expandable introducer 100. In a step 308 of the method 300, the expandable introducer 100 is advanced distally over the guidewire 402 into the vessel 401 of the patient until the distal end 110 of the introducer hub 104 is snug against the vessel 401 of the patient to establish a hemostatic seal, as shown in FIG. 11.

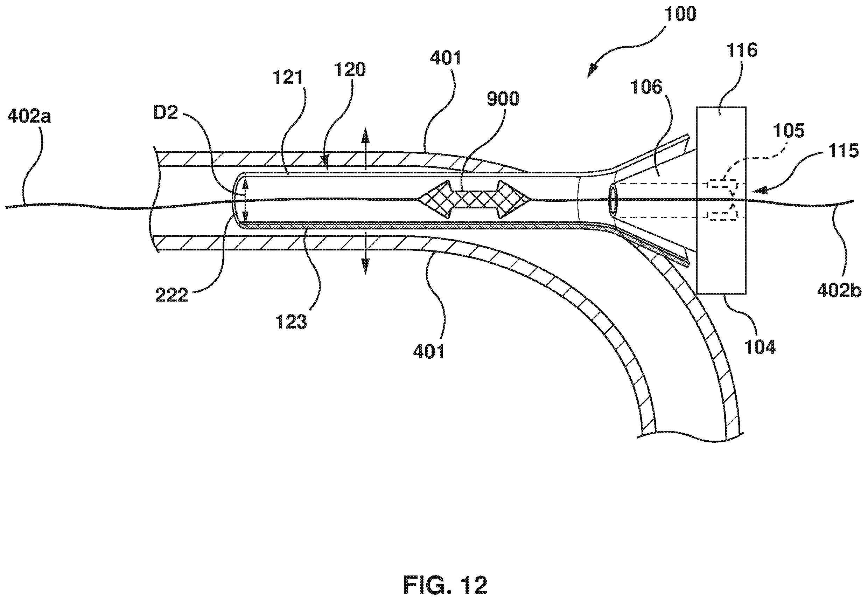

In a step 310 of the method 300, the device 900 may be advanced through the expandable introducer 100. With the distal end 402a of the guidewire 402 disposed within the vessel 401 of the patient, the proximal end 402b of the guidewire 402 may be inserted into a distal end of a central lumen of the device 900. In a step 312 of the method 300, the device 900 may then be distally advanced over the guidewire 402 into the proximal end 108 of the expandable introducer 100 and advanced therethrough, as shown in FIG. 12. As the device 900 is advanced distally through the introducer sheath 120, the central lumen 129 of the introducer sheath 120 radially expands from a folded, unexpanded state having a first diameter D1 to an unfolded, expanded state having a second diameter D2 larger than the first diameter, as shown in FIG. 12. The introducer sheath 120 radially expands via unfolding of the liner 122 and radially expansion of the mesh tube 121, as described above. The device 900 is advanced distally through the central lumen 109 of the expandable introducer 100 and exits through the distal end 107 of the expandable introducer 100, wherein the device 900 can then be tracked over the guidewire 402 until it reaches a desired location within the vasculature of the patient.

Upon the device 900 exiting the introducer sheath 120, the introducer sheath 120 returns to the folded, unexpanded state. The mesh tube 121 may be shape set to the radially unexpanded state such that when the device 900 is not within the central lumen 129, the mesh tube 121 returns to the radially unexpanded state. Similarly, the liner 122 may be shape set to the folded state. In other embodiments, the mesh tube 121 is shape set to the radially collapsed state, and when the mesh tube 121 radially collapses, the mesh tube 121 forces the liner 122 to the folded state.

In some embodiments, the method may include the use of an extension tube 902 and a stopcock 901 in order to inject saline, or other fluids, into the proximal end 108 of the expandable introducer 100. In particular, a proximal end of the extension tube 902 is coupled to a side port 903 of the introducer hub 104. The side port 903 is fluidly coupled to the passageway 115 of the introducer hub 104, which is fluidly coupled to the central lumen 109 of the introducer sheath 200. Therefore, injecting saline, or other fluids, into the extension tube 902 results in the saline or other fluid being injected into the central lumen 129 of the introducer sheath 129. Injecting a fluid into the central lumen 129 of the introducer sheath 120 of the expandable introducer 100 manually expands the introducer sheath 120 to reduce the risk of the device 900 snagging or getting caught on the introducer sheath when the device is advanced distally through the central lumen 129 of the introducer sheath 120. The stopcock 901 is coupled to a distal end of the extension tube 902 and is configured to control the flow of saline, or other fluids, through the extension tube 902 and into the vasculature of the patient.

Those skilled in the art will recognize that other steps may be included and that the order in which the steps are described are not necessarily limiting.

Embodiments hereof are also directed to a method (not claimed) for manufacturing the introducer sheath 200. In a first step, the introducer sheath 200 having the mesh tube 101, the liner 102 disposed inside the interior surface of the mesh tube 101, and the axial strip 103 disposed between the liner 102 and the mesh tube 101, is loaded onto a mandrel. A heat shrink wrap is used to melt the axial strip 103, wherein the axial strip 103 bonds to the liner 102 and interconnects within the mesh tube 101. The introducer sheath 200 is then allowed to cool and harden, and then the introducer sheath 200 may be removed from the mandrel.

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components associated with, for example, a medical device.

## Claims

1. An expandable introducer (100) comprising:
an introducer hub (104); and
an introducer sheath (120) coupled to the introducer hub and extending distally therefrom, the introducer sheath including:
a mesh tube (121);
a liner (122) disposed inside the mesh tube, the liner defining a central lumen; and
an axial strip (123) disposed between the liner and the mesh tube, the axial strip bonding the liner to the mesh tube;
wherein the introducer sheath is radially expandable from a radially unexpanded state wherein the central lumen has a first diameter (D1) to a radially expanded state wherein the central lumen has a second diameter (D2) larger than the first diameter; wherein the liner in the radially unexpanded state includes a fold (124), and wherein the liner unfolds to expand to the radially expanded state.

2. The expandable introducer of claim 1, wherein the first diameter is in the range of about 3 mm to about 5 mm.

3. The expandable introducer of any one of claim 1 or 2, wherein the second diameter is in the range of about 8 mm to about 10 mm.

4. The expandable introducer of any one of claims 1 to 3, wherein the mesh tube comprises a braided, mesh material comprising a plurality of braided fibers arranged in a braided or mesh pattern to form an open-cell fibrous wall material.

5. The expandable introducer of claim 4, wherein the plurality of braided fibers comprise a polymeric material.

6. The expandable introducer of any one of claims 1 to 5, wherein the liner is a thin, flexible plastic tube configured to expand from a folded state to an unfolded state in response to a device being advanced through the central lumen.

7. The expandable introducer of claim 5, wherein the liner comprises a single fold that is configured to unfold to radially expand the liner as a device is advanced through the central lumen.

8. The expandable introducer of claim 5, wherein the liner comprises two or more folds that are configured to unfold to radially expand the liner as a device is advanced through the central lumen.

9. The expandable introducer of any one of claims 1 to 8, wherein the liner comprises polytetrafluoroethylene (PTFE), expanded polytetrafluoroethylene (ePTFE), fluorinated ethylene propylene (FEP), and/or perfluoroalkoxy (PFA).

10. The expandable introducer of any one of claims 1 to 9, wherein the liner has a thickness in the range of about 0.05 mm to about 0.40 mm.

11. The expandable introducer of any one of claims 1 to 10, wherein the axial strip comprises a high durometer polymer; and/or wherein the axial strip comprises a polyamide 12 material and/or a polyether block amide material.

12. The expandable introducer of any one of claims 1 to 11, wherein the axial strip extends about 70° to about 180° around a circumference of the mesh tube with the introducer sheath in the radially unexpanded state.

13. The expandable introducer of any one of claims 1 to 12, wherein the axial strip includes an axial spine (301).

14. The expandable introducer of claim 13, wherein the axial spine comprises a thin, rigid wire; or wherein the axial spine comprises two or more thin, rigid wires.

15. The expandable introducer of any one of claims 1 to 14, wherein the introducer sheath includes a proximal portion coated with a substantially non-porous expandable layer to assist with establishing a hemostatic seal when the expandable introducer is introduced to a patient's vasculature.

## Patentansprüche

1. Erweiterbare Einführvorrichtung (100), umfassend:
eine Einführnabe (104); und
eine Einführschleuse (120), die an die Einführnabe gekoppelt ist und sich distal davon erstreckt, wobei die Einführschleuse einschließt:
einen Gitterschlauch (121);
eine Auskleidung (122), die innerhalb des Gitterschlauchs angeordnet ist, wobei die Auskleidung ein zentrales Lumen definiert; und
einen axialen Streifen (123), der zwischen der Auskleidung und dem Gitterschlauch angeordnet ist, wobei der axiale Streifen die Auskleidung mit dem Gitterschlauch verbindet;
wobei die Einführschleuse radial von einem radial nicht erweiterten Zustand erweiterbar ist, wobei das zentrale Lumen einen ersten Durchmesser (D1) zu einem radial erweiterten Zustand aufweist, wobei das zentrale Lumen einen zweiten Durchmesser (D2) aufweist, der größer als der erste Durchmesser ist;
wobei die Auskleidung in dem radial nicht erweiterten Zustand eine Falte (124) einschließt, und wobei sich die Auskleidung entfaltet, um sich in den radial erweiterten Zustand zu erweitern.

2. Erweiterbare Einführvorrichtung nach Anspruch 1, wobei der erste Durchmesser in dem Bereich von etwa 3 mm bis etwa 5 mm liegt.

3. Erweiterbare Einführvorrichtung nach einem der Ansprüche 1 oder 2, wobei der zweite Durchmesser in dem Bereich von etwa 8 mm bis etwa 10 mm liegt.

4. Erweiterbare Einführvorrichtung nach einem der Ansprüche 1 bis 3, wobei der Gitterschlauch ein geflochtenes Gittermaterial umfasst, umfassend eine Vielzahl von geflochtenen Fasern, die in einem geflochtenen oder Gittermuster eingerichtet sind, um ein offenzelliges faseriges Wandmaterial auszubilden.

5. Erweiterbare Einführvorrichtung nach Anspruch 4, wobei die Vielzahl von geflochtenen Fasern ein Polymermaterial umfassen.

6. Erweiterbare Einführvorrichtung nach einem der Ansprüche 1 bis 5, wobei die Auskleidung ein dünner, flexibler Kunststoffschlauch ist, der konfiguriert ist, um sich von einem gefalteten Zustand zu einem entfalteten Zustand als Reaktion darauf zu erweitern, dass eine Vorrichtung durch das zentrale Lumen vorgeschoben wird.

7. Erweiterbare Einführvorrichtung nach Anspruch 5, wobei die Auskleidung eine einzelne Falte umfasst, die konfiguriert ist, um sich zu entfalten, um die Auskleidung radial zu erweitern, wenn eine Vorrichtung durch das zentrale Lumen vorgeschoben wird.

8. Erweiterbare Einführvorrichtung nach Anspruch 5, wobei die Auskleidung zwei oder mehrere Falten umfasst, die konfiguriert sind, um sich zu entfalten, um die Auskleidung radial zu erweitern, wenn eine Vorrichtung durch das zentrale Lumen vorgeschoben wird.

9. Erweiterbare Einführvorrichtung nach einem der Ansprüche 1 bis 8, wobei die Auskleidung Polytetrafluorethylen (PTFE), erweitertes Polytetrafluorethylen (ePTFE), fluoriertes Ethylenpropylen (FEP) und/oder Perfluoralkoxy (PFA) umfasst.

10. Erweiterbare Einführvorrichtung nach einem der Ansprüche 1 bis 9, wobei die Auskleidung eine Dicke in dem Bereich von etwa 0,05 mm bis etwa 0,40 mm aufweist.

11. Erweiterbare Einführvorrichtung nach einem der Ansprüche 1 bis 10, wobei der axiale Streifen ein Polymer mit hohem Härtegrad umfasst; und/oder wobei der axiale Streifen ein Polyamid-12-Material und/oder ein Polyetherblockamid-Material umfasst.

12. Erweiterbare Einführvorrichtung nach einem der Ansprüche 1 bis 11, wobei sich der axiale Streifen etwa 70° bis etwa 180° um einen Umfang des Gitterschlauchs erstreckt, wobei sich die Einführschleuse in dem radial nicht erweiterten Zustand befindet.

13. Erweiterbare Einführvorrichtung nach einem der Ansprüche 1 bis 12, wobei der axiale Streifen ein axiales Rückgrat (301) einschließt.

14. Erweiterbare Einführvorrichtung nach Anspruch 13, wobei das axiale Rückgrat einen dünnen, starren Draht umfasst; oder wobei das axiale Rückgrat zwei oder mehrere dünne, starre Drähte umfasst.

15. Erweiterbare Einführvorrichtung nach einem der Ansprüche 1 bis 14, wobei die Einführschleuse einen proximalen Abschnitt einschließt, der mit einer im Wesentlichen nicht porösen erweiterbaren Schicht beschichtet ist, um bei einem Etablieren einer hämostatischen Abdichtung zu unterstützen, wenn die erweiterbare Einführvorrichtung in ein Gefäßsystem eines Patienten eingeführt wird.

## Revendications

1. Dispositif introducteur expansible (100) comprenant :
un moyeu de dispositif introducteur (104) ; et
une gaine de dispositif introducteur (120) accouplée au moyeu de dispositif introducteur et s'étendant distalement à partir de celui-ci, la gaine de dispositif introducteur comportant :
un tube en maille (121) ;
un revêtement (122) disposé à l'intérieur du tube en maille, le revêtement définissant une lumière centrale ; et
une bande axiale (123) disposée entre le revêtement et le tube en maille, la bande axiale liant le revêtement au tube en maille ;
dans lequel la gaine de dispositif introducteur est radialement expansible d'un état radialement non expansé dans lequel la lumière centrale a un premier diamètre (D1) à un état radialement expansé dans lequel la lumière centrale a un second diamètre (D2) supérieur au premier diamètre ;
dans lequel le revêtement à l'état radialement non expansé comporte un pli (124), et dans lequel le revêtement se déplie pour s'expanser à l'état radialement expansé.

2. Dispositif introducteur expansible selon la revendication 1, dans lequel le premier diamètre est compris dans la plage d'environ 3 mm à environ 5 mm.

3. Dispositif introducteur expansible selon l'une quelconque des revendications 1 ou 2, dans lequel le second diamètre est dans la plage d'environ 8 mm à environ 10 mm.

4. Dispositif introducteur expansible selon l'une quelconque des revendications 1 à 3, dans lequel le tube en maille comprend un matériau en maille tressé comprenant une pluralité de fibres tressées agencées selon un motif tressé ou en maille pour former un matériau de paroi fibreuse à cellules ouvertes.

5. Dispositif introducteur expansible selon la revendication 4, dans lequel la pluralité de fibres tressées comprennent un matériau polymère.

6. Dispositif introducteur expansible selon l'une quelconque des revendications 1 à 5, dans lequel le revêtement est un tube en plastique mince et souple conçu pour s'expanser d'un état plié à un état déplié en réponse à l'avancement d'un dispositif dans la lumière centrale.

7. Dispositif introducteur expansible selon la revendication 5, dans lequel le revêtement comprend un pli unique qui est conçu pour se déplier afin d'expanser radialement le revêtement en réponse à l'avancement d'un dispositif dans la lumière centrale.

8. Dispositif introducteur expansible selon la revendication 5, dans lequel le revêtement comprend deux ou plusieurs plis qui sont conçus pour se déplier afin d'expanser radialement le revêtement en réponse à l'avancement d'un dispositif dans la lumière centrale.

9. Dispositif introducteur expansible selon l'une quelconque des revendications 1 à 8, dans lequel le revêtement comprend du polytétrafluoroéthylène (PTFE), du polytétrafluoroéthylène expansé (ePTFE), de l'éthylène propylène fluoré (FEP) et/ou du perfluoroalcoxy (PFA).

10. Dispositif introducteur expansible selon l'une quelconque des revendications 1 à 9, dans lequel le revêtement a une épaisseur dans la plage d'environ 0,05 mm à environ 0,40 mm.

11. Dispositif introducteur expansible selon l'une quelconque des revendications 1 à 10, dans lequel la bande axiale comprend un polymère à haute dureté ; et/ou dans lequel la bande axiale comprend un matériau polyamide 12 et/ou un matériau polyéther bloc amide.

12. Dispositif introducteur expansible selon l'une quelconque des revendications 1 à 11, dans lequel la bande axiale s'étend d'environ 70° à environ 180° autour d'une circonférence du tube en maille avec la gaine de dispositif introducteur dans l'état radialement non expansé.

13. Dispositif introducteur expansible selon l'une quelconque des revendications 1 à 12, dans lequel la bande axiale comporte une colonne axiale (301).

14. Dispositif introducteur expansible selon la revendication 13, dans lequel la colonne axiale comprend un fil mince et rigide ; ou dans lequel la colonne axiale comprend deux ou plusieurs fils minces et rigides.

15. Dispositif introducteur expansible selon l'une quelconque des revendications 1 à 14, dans lequel la gaine de dispositif introducteur comporte une partie proximale recouverte d'une couche expansible sensiblement non poreuse pour aider à établir un joint hémostatique lorsque le dispositif introducteur expansible est introduit dans le système vasculaire d'un patient.
